Europäisches Patentamt

European Patent Office

Office européen des brevets

(11) Veröffentlichungsnummer: **0 262 451 B1**

# EUROPÄISCHE PATENTSCHRIFT

(45) Veröffentlichungstag der Patentschrift: 24.04.91

(51) Int. Cl.⁵: **A61L 2/06**, D06F 58/10, D06F 58/20

(21) Anmeldenummer: 87112939.1

(22) Anmeldetag: 04.09.87

(54) Verfahren zum Trocknen und Sterilisieren von Gut im geschlossenen Kreislauf.

(30) Priorität: 26.09.86 DE 3632820

(43) Veröffentlichungstag der Anmeldung:
06.04.88 Patentblatt 88/14

(45) Bekanntmachung des Hinweises auf die
Patenterteilung:
24.04.91 Patentblatt 91/17

(84) Benannte Vertragsstaaten:
AT BE CH DE ES FR GB GR IT LI LU NL SE

(56) Entgegenhaltungen:
EP-A- 0 148 385
EP-A- 0 170 728
DE-B- 1 105 562
DE-C- 959 851
GB-A- 1 133 098

(73) Patentinhaber: **Baltes, Hans**
**Heideweg 27**
**W-4600 Dortmund(DE)**

(72) Erfinder: **Baltes, Hans**
**Heideweg 27**
**W-4600 Dortmund(DE)**

(74) Vertreter: **Schulze Horn, Stefan, Dipl.-Ing.**
**M.Sc.**
**Goldstrasse 36**
**W-4400 Münster(DE)**

## Beschreibung

Die Erfindung betrifft ein Verfahren und eine Vorrichtung zum Trocknen und Sterilisieren, insbesondere von Wäsche, besonders empfindlichen Geweben, reine Wolle, Oberbetten, Kissen, Daunendecken, Wolldecken und Leder und beliebigen weiteren Gegenständen, z. B. Bestecke, medizinische Instrumente, Kämme, Scheren, Bursten, Messer, Rasierapparate, in einem Trocken- und Sterilisationsschrank, wobei sowohl die Trocknung als auch die Sterilisation der z. B. Gewebe im gleichen Behandlungsraum in stehendem oder in hängendem, mechanisch unbewegten Zustand durchgeführt und die Sterilisation unmittelbar im Anschluß an die Trocknung vorgenommen wird.

Es sind verschiedene Methoden zur Trocknung und Sterilisation bekannt. Trommeltrockner, in verschiedenen Varianten auftretend, sind in der Lage, bei unterschiedlichen Temperaturen Gewebe zu trocknen. Hierbei ist das ständige Bewegen des Trockengutes zugleich verbunden mit einer Zufuhr von erwärmter Luft. Neben diesen Trommeltrocknern existieren auch Trockenschränke, in denen das hängende Wäschegut der Luft ausgesetzt ist, die entweder erwärmt ist oder Zimmertemperatur besitzt.

Die Sterilisation des Wäscheguts selbst erfolgt normalerweise nicht in den Trockengeräten, sondern hierfür sind weitere Verfahren notwendig. So sind aus der Hygiene und Mikrobiologie unterschiedliche Sterilisationsverfahren bekannt, wobei die für die Sterilisation von Wäschegut relevanten Verfahren nur aufgezählt werden. Das Autoklavieren bei 121° C und 2 bar Dampfdruck ist eine vielbenutzte Technik, um temperaturempfindliche Stoffe keimfrei zu machen. Dieses Verfahren wird vor allen Dingen dann verwandt, wenn Temperaturen von 160° C und mehr dem Material nicht mehr zuzumuten sind, ohne daß Schäden eintreten. Um bei Zimmertemperatur dennoch eine Sterilisation vorzunehmen, kann das Sterilisationsgut Gasen ausgesetzt werden, wie z. B. Ethylenoxid, Formaldehyd, Chlor, Ozon usw., oder es läßt sich auch in wässrigem Medium durch z. B. Oxidationsmittel oder Aldehydgruppen enthaltende Desinfektionsmittel (Formaldehyd) das Sterilisationsgut entkeimen. Dabei muß man beachten, daß das chemische Verfahren immer sehr langwierig ist. Weiterhin stehen zur Sterilisation sehr empfindlicher Guter auch die Bestrahlung mit Gammastrahlen zur Verfugung, eine Bestrahlung mit Röntgenstrahlen wurde in der Dosisleistung nicht ausreichen.

Bei den heutigen Waschverfahren bei 30, 60 und 90° C ist grundsätzlich davonauszugehen, daß ein Kochen der Wäsche nicht mehr vorgenommen wird. Zugleich ist auch zu bedenken, daß viele Gewebearten derart empfindlich sind, daß ein Waschen bei höheren Temperaturen zu einer Schädigung des Materials fuhren würde. Bei allen heutigen Waschvorgängen findet zugleich auch eine starke mechanische Belastung statt, die zugleich auch die Höhe der Waschtemperatur begrenzt. Aus denselben Gründen können Trommeltrockner bei empfindlichen Geweben nicht höhere Temperaturen benutzen, als etwa 50 - 60° C, da sonst bei der thermischen und mechanischen Belastung des Gewebes dauerhafte Schäden in kurzer Zeit auftreten.

Hieraus wird ersichtlich, daß bei den heutigen Waschprozessen und einem anschließenden Trocknen in Trommeltrocknern eine Reinigung sehr wohl möglich, jedoch eine anschließende Sterilisation des Wäscheguts nicht durchführbar ist. Man muß daher davon ausgehen, daß das trokkene Wäschegut anschließend durchaus noch eine Reihe von Keimen aufweist.

Daher ist es bei Wäschestücken, Kleidungsstucken notwendig, anschließend noch einen Sterilisationsvorgang vorzunehmen. Da Gewebe bei hohen Temperaturen, d. h. 160° C, geschädigt werden, muß das Gut durch Autoklavieren, durch chemische Substanzen oder durch Strahlung sterilisiert werden. Das Arbeiten mit dem Autoklaven darf durchaus nicht fur den technisch nicht ausgebildeten Laien als ungefährlich angesehen werden. Hier hat man es einerseits mit hohen Temperaturen, 121° C, zu tun, so daB einige Teile des Gerätes diese Temperatur aufweisen, andererseits muß ein Druck von 2 bar aufgebaut werden, was eine Druckkammer voraussetzt, die ein kompliziertes Öffnungssystem besitzen muB. Ein Autoklav setzt weiterhin eine hohe technische Wartung voraus, die die Kosten dieses Systems erheblich steigern.

Wäschestucke lassen sich aber auch durch chemische Methoden sterilisieren. Dabei ist zu bedenken, daß man es mit Substanzen zu tun hat, die zugleich auch fur die Haut der späteren Träger negativ sich auswirken können, dann nämlich, wenn diese Substanzen nicht völlig wieder aus den Geweben entfernt worden sind. So können höhermolekulare Substanzen in den Wäschestücken verbleiben und hinterher bei den Trägern Abwehrreaktionen, z. B. Allergien, hervorrufen. Doch nicht nur für den Träger ergeben sich durch diese chemischen Substanzen Schwierigkeiten, sondern auch bei deren Beseitigung. Handelt es sich um Gase, werden dieselbigen in die Umluft freigesetzt, bei flüssigen Substanzen wird das Abwasser belastet. Sowohl eine Verunreinigung der Luft als auch der Gewässer ist im hohen Maße zu vermeiden. Auch die Sterilisation mit GammaStrahlen stellt ein gewisses Problem dar. Zwar werden durch diese Behandlung die Keime getötet, ohne daß das Sterilisationsgut erhitzt oder chemischen Substanzen ausgesetzt werden müßte, aber man muß bedenken, daß eine derartige Strahlendosis nicht durch Röntgengeräte erreicht werden, sondern allein nur durch natürliche Radionukleotide bewirkt werden kann. Dadurch ergibt sich auch hier neben dem Problem der Anlagenkosten das Problem der Entsorgung.

Der Erfindung liegt die Aufgabe zugrunde, in besonders schlichter Art eine Trocknung und Sterilisation durchzuführen, dabei die Trocken- und Sterilisationszeit bei vertretbarem Kostenaufwand zu minmieren, umweltschädliche gasförmige oder wasserlösliche Substanzen zu vermeiden, die Handhabung in schlichter und einfacher Art und Weise auch für den technisch nicht vorgebildeten Benutzer sicher zu machen und die Luft, die beim Trocknungsvorgang und bei der Sterilisation mit den Wäscheteilen in Kontakt gekommen ist, in einem geschlossenen Kreislauf zu fuhren, so daß eine Kontamination der Umgebung ausgeschlossen ist.

Diese Aufgabe wird erfindungsgemäß dadurch gelöst, daß der Luftstrom in einem geschlossenen Kreislauf bestehend aus einem inneren und einem äußeren Kreislauf gefuhrt wird und daB durch ein Kondensations- und Kühlaggregat beide Systeme miteinander energiemäßig in Kontakt stehen, daß die Kondensation mittels des Kondensations-und Kühlaggregats nach Ende der Trocknung eingestellt wird und daß nach der Sterilisation das Kondensations-und Kühlaggregat abgekühlt wird. Bei dem Trocknen und Sterilisieren kann die Kondensation bei Warmlufttemperatur unter 100° C, d. h. bei 45 bis 60° C, und die Sterilisation bei Heißlufttemperatur im Bereich zwischen 121 und 130° C, vorzugsweise zwischen 125 und 130° C, vorgenommen werden.

Fur den Fachmann völlig unerwartet ist, daß eine vollständige Sterilisation bei Temperaturen möglich ist, die entsprechend der Erfindung benutzt werden. Daß eine Sterilisation bei diesen Temperaturen durchfuhrbar ist, widerspricht sogar dem Stand der Technik, da es als erwiesen gilt, daß eine Trocken-Sterilisation nur bei Temperaturen von 160° C und mehr möglich ist.

Um die tatsächlich durchgeführte Sterilisation zu belegen, wurden unterschiedliche Tests durchgeführt. Hierfür wurde in ein Modell eines Trocken- und Sterilisationsschrankes gemäß der Erfindung an unterschiedlichen Stellen kontaminiertes Material gelegt. Als Testkeime wurden Staphelococcus aureus, Pseudomonas aeroginosa, Bacillus subtilis und Candida albicans verwandt. Hierbei handelt es sich um Keime, die überall verbreitet sind und die bis auf Bacillus subtilis alle Problemkeime innerhalb der Krankenhäuser sind, da diese Keime wegen ihrer Antibiotika-Resistenz schwer zu bekämpfen sind. Diese Testkeime wurden in einem Vollmedium angezüchtet und in hoher Konzentration auf das Gewebe aufgetragen. Dabei wurden zum einen die Testkeime mit Vollmedium, zum anderen mit Blut vermischt. Diese Beimischung, d. h. das Vollmedium und das Blut, gewähren normalerweise den Keimen einen Schutzbereich, der es ihnen erlaubt, extremen Umweltbedingungen zu widerstehen. In dem Schrank gemäß der Erfindung wurde das kontaminierte Material einer Temperatur von reel 125 - 130° C ausgesetzt. Anschließend wurde das Material unter sterilen Bedingungen dem sterilen Kulturmedium, einem Vollmedium, ausgesetzt. Die Inkubationszeit beträgt 8 Tage bei 37°, so daB allein schon ein überlebender Keim ausreicht, um ein deutliches Wachstum in dem Kulturmedium nachzuweisen. Das Ergebnis dieser Testserien zeigt, daß eine effektive Sterilisationszeit von 30 Minuten ausreichend ist für all die Keime, die keine Endosporen ausbilden. Für die erfolgreiche Sterilisation auch dieser Endosporen-Bildner sind 120 Minuten Sterilisationszeit ausreichend. Mit einem Gerät gemäß der Erfindung ist daher eine sichere Sterilisation möglich, wenn bei Temperaturen zwischen 125 - 130° C das Sterilisationsgut über 120 Minuten dieser Temperatur ausgesetzt ist.

Derartige Bedingungen sind neu für die Fachwelt.

Bei einem Trocken- und Sterilisationsschrank dürfen Keime, die sich auf dem zu trocknenden Gewebe befinden, nicht in die Umwelt freigesetzt werden. Dieses Problem ist durch einen geschlossenen, internen Kreislauf zu lösen.

Normalerweise sind Gewebe nicht Temperaturen auszusetzen, die bei 125 - 130° C liegen. Das Arbeiten bei diesen Temperaturen ist möglich, da verschiedene Bedingungen erfüllt werden. Zum einen läuft die Trockenphase bei vorzugsweise 40 - 65° C ab, zum anderen wird während der Heißphase von 121 - 130° C das Gewebe nicht mechanisch belastet.

Durch diese Erfindung werden mehrere Vorteile gegenüber dem alten Stand der Technik erreicht.

So können sämtliche Gewebe, wie Mischgewebe, Baumwolle, reine Wolle, reine Seide, reine Synthetics und reine Viskose sowohl schonend getrocknet als auch sterilisiert werden. Weiterhin können derselben Behandlung z. B. Leder, Leder-Schuhe oder auch Federbetten unterzogen werden, was einer Hygiene auf diesen Gebieten sehr dienlich ist. Bei dem TrocknungsprozeB kommt ein sehr schonendes Verfahren zur Anwendung, da nicht mit einem Verdampfen gearbeitet wird, sondern lediglich ein Verdunsten des Wassers stattfindet, d. h. die Trocknung findet unterhalb der Siedetemperatur des Wassers bei Normaldruck statt.

Die Wäsche wird in dem Trocken- und Sterilisationsschrank in hängender Form bearbeitet, dadurch wird im Gegensatz zu den Trommeltrocknern kein Abrieb, kein Knittern, kein Verschleiß der Wäsche herbeigeführt. Ein Verfilzen, ein Verformen, ein Verfärben und ein Einlaufen der Wäscheteile findet nicht statt, so daß letztendlich die Wäsche länger hält und für den Benutzer sich als Kostenfaktor bemerkbar macht.

Nach dem Trocknen liegt die Wäsche schon in glatter Form vor, so daß eine Weiterbehandlung kaum nötig ist, was ebenfalls zu einer Kostenreduktion führt.

Im Gegensatz zum Trommeltrockner benötigt dieses Gerät weniger Platz. Da eine dem Trommeltrockner vergleichbare Mechanik entfällt, ist ebenfalls eine Anbringung weit problemloser. Da es sich lediglich um Gebläse handelt, die in Bewegung sind, ist eine Schallbelastung, die sich z. B. durch die Wände oder den Fußboden fortpflanzt, ausgeschlossen.

Da bei dem Gerät keine Brandgefahr besteht, kann es auch ohne Beaufsichtigung betrieben werden. Da das Gerät gemäß der Erfindung auf Einzelteile einer ausgereiften Technik zurückgreift, wird ein hoher Grad an Zuverlässigkeit erreicht, so daß mit einem hohen Interesse sowohl vom Krankenhaussektor als auch vom Hotelgewerbe zu rechnen ist. Fur Krankenhäuser und Kliniken ergibt sich mit Hilfe dieses Trocken- und Sterilisationsschrankes eine gute Möglichkeit, die Dienstkleidung, medizinische Instrumente, Bestecke und Geschirr, weiterhin Handtucher, Bettzeug und Kleidungsstücke zu sterilisieren. Hierbei braucht nicht mehr auf kostspieligere Geräte oder auf chemische Substanzen zurückgegriffen werden, die bisweilen nur zu einer Desinfektion führen und die darüber hinaus auch die Umwelt hochgradig belasten, sondern es bietet sich an, daß Sterilgut in technisch schlichter Art und Weise zu sterilisieren. Fur die Krankenhäuser muß ein solches Gerät allein deswegen von hohem Interesse sein, da ein Teil von Infektionen gerade durch das Krankenhauspersonal verbreitet wird, und es zu den sogenannten Hospitalismus-Infektionen kommen kann. Diese Keime mit Medikamenten oder chemischen Stoffen zu bekämpfen, muß als sehr problematisch angesehen werden, da diese Keime sich durch eine besondere Resistenz auszeichnen. Jedoch gegenüber einer Sterilisation durch Temperatur sind diese Keime empfindlich.

Ebenso ist es fur Frisöre von Bedeutung, Kämme, Scheren, Bürsten, Messer und Rasierapparate gemäß dem erfinderischen Verfahren sterilisieren zu können.

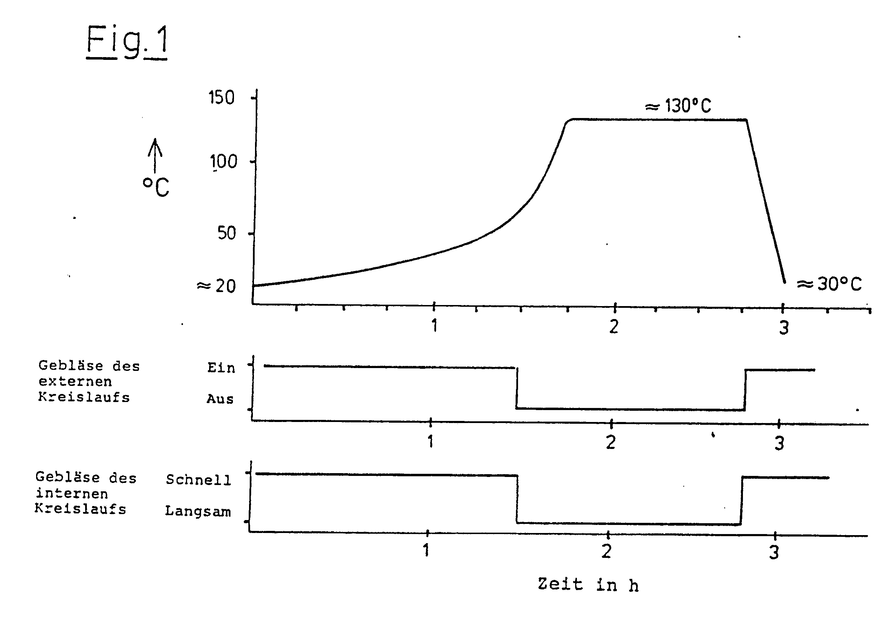

Um exemplarisch einen Prozeßverlauf darzustellen, wurde eine Grafik in der Figur 1 gewählt. Die Wäsche kommt bei Raumtemperatur in den Trocken- und Sterilisationsschrank und wird innerhalb von 1 1/2 Stunden getrocknet. Dabei steigt die Temperatur allmählich bis etwa 65° an. Die Feuchtigkeit wird mittels des Kondensations- und Kühlaggregat aus dem inneren Luftkreislauf entfernt. Beträgt die Luftfeuchtigkeit etwa 65 %, schaltet das Gerät um, so daß der innere Luftstrom nur noch langsamer bewegt wird, der äußere Kühlkreislauf wird vollständig abgestellt. Dadurch erhöht sich die Innentemperatur auf etwa 125 -130° C, dieses geschieht in etwa 15 Minuten.

Erst danach findet die tatsächliche effektive Sterilisationsphase statt. Sie verläuft über eine Stunde. Erst danach findet die tatsächliche effektive Sterilisationsphase statt. Sie verläuft über eine Stunde. Während dieser Zeit wird die Temperatur innerhalb des Schrankes über einen Regelkreis konstant gehalten, der aus zum Innenraum in Kontakt stehenden Temperatursensoren, einem Sollwertgeber und einem ein- und abstellbaren Heizelement besteht. Durch die während der Sterilisation erfolgende Luftumwälzung ist auch bei dem Heizelement, das sich außerhalb der Innenwandung, aber innerhalb des inneren Luftstromes befindet, gewährleistet, auf kleinere Temperaturschwankungen im Innenraum korrigierend zu reagieren.

Im Anschluß an die Sterilisation wird das Sterilgut wieder abgekühlt. Dieses wird dadurch erreicht, daß der interne Luftstrom wieder auf voller Leistung läuft und die externe Kühlung ebenfalls wieder eingeschaltet wird. Dadurch ist innerhalb kurzer Zeit die Reduktion der Temperatur um etwa 90 - 100° zu erreichen. Die Wäsche und die Innenteile des Schranks sind nach dieser Abkuhlphase etwa 30 - 40° C warm, so daß jede Gefahr fur das Bedienungspersonal ausgeschlossen ist. Nach diesem Prozeß ist der Trocken- und Sterilisationsschrank wieder voll einsatzfähig.

Vorteilhafterweise kann die Innenwandung des Trocken- und Sterilisationsschrankes aus wärmeisolierendem Material bestehen. Dabei soll das Material gegenüber einem Wechsel der Luftfeuchtigkeit unempfindlich sein. Im Hinblick auf die Verwendung in einem Trocken- und Sterilisationsschrank ist es erforderlich, daß die Innenwände durch einen schnellen Wechsel von feuchter Hitze und extremer Trockenheit nicht in Mitleidenschaft gezogen werden.

Weiterhin garantiert ein wärmeisolierendes Material, daß an Berührungsstellen von Textilien und Schrankinnenwandung oder auch der Halterung mögliche Gilbbildung an den Textilien nicht auftritt. Bei einigen empfindlichen Textilien ist nicht mit Sicherheit auszuschließen, daß sich bei Kontakt mit Metallflächen und Metallwandungen eine derartige Vergilbung ergibt.

Derartig wärmeisolierendes Material führt weiterhin dazu, daß das Bedienungspersonal keine Verbrennungen erleidet, wenn nach Öffnung des Schrankes während der Sterilisationsphase zufällig die Innenflächen des Trocken- und Sterilisationsschrankes mit der bloßen Hand berührt werden. Weiterhin besitzt dieses isolierende Material den großen Vorteil, daß wegen der geringen Wärmeaufnahme und der extremen Wärmeisolation die Auf- und Abkuhlphase schnell und mit geringem Energieaufwand erfolgen kann.

Besteht zu der Innenwandung ebenfalls auch die äußere Wandung des Trocken- und Sterilisationsschrankes aus einem isolierenden Material, ist eine gute Isolierung auch gegenüber der Umgebung gewährleistet. Der Vorteil eines solchen Schrankes besteht darin, daß zwischen der hohen Hitze im Inneren und der Umgebungstemperatur eine gute Isolierung ermöglicht wird. Dieses hat zur Folge, daß Energie beim Aufheizen gespart werden kann, die Abkuhlphase schnell erfolgt und eine umständliche und raumverschwendende Abschirmung gegenüber umliegenden Möbelstücken und Wandungen nicht vorgenommen werden muß. Ein derartiger Schrank könnte somit direkt an ein anderes Gerät oder an eine Wand räumlich anschließen, ohne daß ein Luftraum zur Abführung der Wärme und zur Isolierung vorhanden sein muß.

Um die Abkühlung zu beschleunigen, kann als weitere Möglichkeit ein Teil der Kühlluft über die Außenwände (z. B. Seiten-, Decken- und/oder Rukkenwandkanäle) angesaugt werden, um diese zu kuhlen.

Die Erfindung wird in einer Zeichnung in einer bevorzugten Ausführungsform gezeigt, wobei aus der Zeichnung weitere vorteilhafte Einzelheiten der Erfindung entnehmbar sind. Die Zeichnung zeigt im einzelnen:

Figur 2:  Seitenansicht eines Trocken- und Sterilisationsschrankes als Schnittbild mit der Perspektive aus der Vorderansicht,

Figur 3:  Grundriß des Trocken- und Sterilisationsschrankes im unteren Teilbereich, in dem die Gebläse und das Kondensations- und Kühlaggregat sich befindet,

Figur 4:  Seitenansicht des Trocken- und Sterilisationsschranks als Schnittbild mit der Schnittebene C, D,

Figur 5:  Seitenansicht des Trocken- und Sterilisationsschranks als Schnittbild mit der Schnittebene A, B,

Figur 6:  perspektivische Darstellung des Kondensationsund Kühlaggregaten.

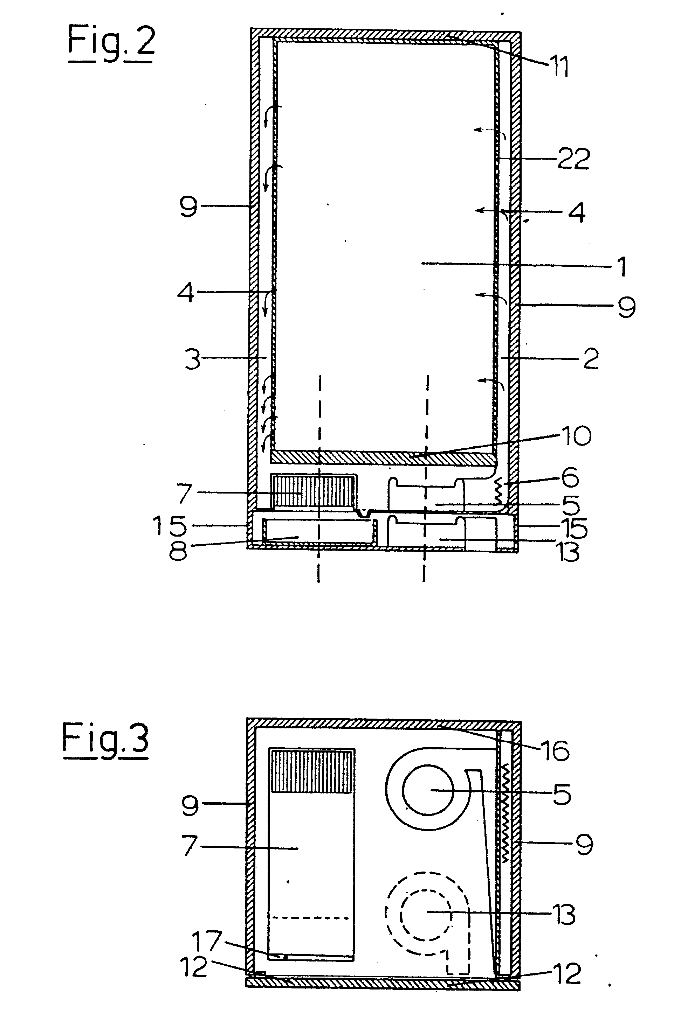

Die Figur 2 zeigt einen Trocken- und Sterilisationsschrank im Schnitt von der Vorderseite aus betrachtet. Ein Schranklumen 1 weist an inneren Seitenwänden 22 Bohrungen 4 auf, durch die sowohl die Luft in das Lumen eintritt als auch das Lumen verläßt. Durch eine Luftzufuhr 2 fur das Schranklumen tritt die Luft in das Lumen ein und verläßt den Innenraum durch die Öffnungen der Bohrungen 4, um durch einen Luftabfuhrkanal 3 zu einem Kondensations-und Kühlaggregat 7 zu gelangen. Nach dem Durchlaufen des Kondensations- und Kühlaggregats 7 wird der Luftstrom von einem Radialgebläse 5 für den inneren Kreislauf angesaugt und anschließend an Heizelementen 6 vorbeigefuhrt, um den Kreislauf des inneren Systems zu schließen. Unter dem Kondensations- und Kühlaggregat 7 befindet sich eine Tropfschale 8, beide sind, wie aus Figur 3 hervorgeht, durch eine Abflußöffnung 17 miteinander verbunden.

Um eine gute Wärmeisolierung zu garantieren, sind die inneren Seitenwände 22, die äußeren Seitenwände 9, die Rückwand 16, die Tür 12, die Decke 11 und der Innenboden 10 wärmeisoliert.

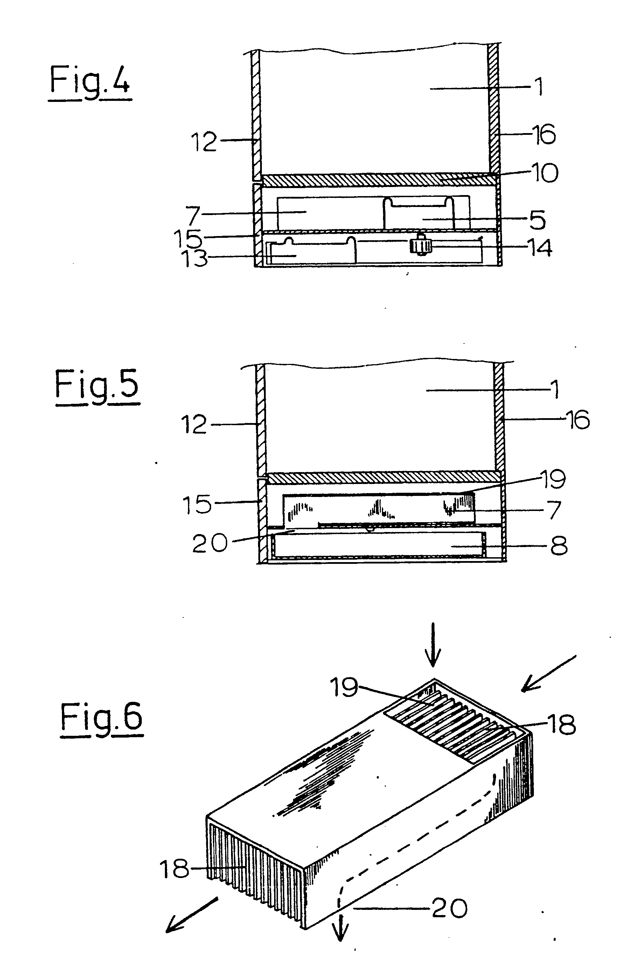

Um das Kondensations- und Kühlaggregat 7 entsprechend zu kühlen, wird innerhalb des externen, offenen Kreislaufs Luft von außerhalb angesaugt, durch das Kondensationsund Kühlaggregat 7 geführt und von einem Radialgebläse 13 fur den externen Kreislauf aufgenommen und von dem Gebläse 13 aus dem Schrank wieder ausgestoßen. Wie in der Figur 6 ersichtlich ist, wird die externe kalte Luft durch eine Einlaßöffnung 19 in das Kondensations- und Kuhlaggregat angesaugt an Lamellen 18 des Kondensations-und Kühlaggregats vorbeigeführt, um an einem Auslaß 20 des Kondensators dieses Aggregat zu verlassen. Da das Kondensations- und Kühlaggregat als Gegenstrom-Kondensator arbeitet, wird die kalte externe Luft beim Vorbeistreichen an den Lamellen 18 erwärmt.

Um eine übermäßige Erwärmung eines Motors 14, der das Radialgebläse 5 fur den inneren Kreislauf antreibt, zu vermeiden, ist dieser Motor 14 außerhalb des internen Kreislaufs placiert.

Alle Aggregate, d. h. die Radialgebläse 5 und 13, das Kondensations- und Kühlaggregat 7 und die Tropfschale 8 befinden sich in einem Sockel 15, was aus den Figuren sowohl in Seiten- als auch in Aufsicht zu sehen ist.

Es ist natürlich auch möglich, einen Teil oder auch alle Anordnungen im oberen Teil des Schrankes zu placieren, wenn dieses gewünscht wird.

Bezugszeichenliste:

1 Schranklumen
2 Luftzufuhr
3 Luftabfuhrkanal
4 Bohrung
5 Radialgebläse
6 Heizelement
7 Kondensations- und Kühlaggregat
8 Tropfschale
9 äußere Seitenwand
10 Innenboden

11 Decke

12 Tür
13 Radialgebläse
14 Motor
15 Sockel
16 Rückwand
17 Abflußöffnung
18 Lamellen
19 Einlaßöffnung
20 Auslaß

22 Seitenwand

**Ansprüche**

1. Verfahren zum Trocknen und Sterilisieren von Gut, insbesondere von Wäsche, besonders empfindlichen Geweben, reine Wolle, Oberbetten, Kissen, Daunendecken, Wolldecken und Leder und beliebigen weiteren Gegenständen, z. B. Bestecke, medizinische Instrumente, Kämme, Scheren, Bürsten, Messer, Rasierapparate, in einem Trokken-und Sterilisationsschrank, wobei sowohl die Trocknung als auch die Sterilisation der z. B. Gewebe im gleichen Behandlungsraum in stehendem oder in hängendem, in mechanisch unbewegten Zustand durchgeführt und die Sterilisation unmittelbar im Anschluß an die Trocknung vorgenommen wird,
dadurch gekennzeichnet,
daß der Luftstrom in einem geschlossenen Kreislauf bestehend aus einem inneren und in einem äußeren Kreislauf geführt wird und daß durch ein Kondensations- und Kühleraggregat beide Systeme miteinander energiemäßig in Kontakt stehen, daß die Kondensation mittels des Kondensations-und Kühlaggregat nach Ende der Trocknung eingestellt wird und daB nach der Sterilisation das Kondensations- und Kühlaggregat kühlt.

2. Verfahren nach Anspruch 1, dadurch gekennzeichnet, daß die Kondensation bei Warmlufttemperatur unter 100°C und die Sterilisation bei Heißlufttemperatur im Bereich zwischen 121 und 130° C, vorzugsweise zwischen 125 und 130°C, vorgenommen wird.

3. Verfahren nach Anspruch 1 oder 2, dadurch gekennzeichnet, daß die Kondensation bei einer Temperatur von 45 bis 60°C vorgenommen wird.

4. Verfahren nach einem der Ansprüche 1 bis 3, dadurch gekennzeichnet, daß während der Sterilisationsphase der Luftstrom des inneren Kreislaufes stark verringert wird.

5. verfahren nach einem der Ansprüche 1 bis 3, dadurch gekennzeichnet, daß während der Sterilisationsphase der Luftstrom des äußeren Kreislaufes nicht bewegt wird.

6. Trocken- und Sterilisationsschrank zur Durchführung eines der verfahren nach einem der Ansprüche 1 bis 5, dadurch gekennzeichnet, daß der Trockenschrank zwei durch Elektromotoren getrennt angetriebene Gebläse, eines für den inneren Trocken- und Sterilisationskreislauf und eines für den äußeren Kühlkreislauf, besitzt.

7. Trocken- und Sterilisationsschrank nach Anspruch 6, dadurch gekennzeichnet, daß die Innenwandung aus einem wärmeisolierenden Material besteht.

8. Trocken- und Sterilisationsschrank nach Anspruch 6, dadurch gekennzeichnet, daß der gesamte Innenraum mit einer Isolationsschicht ausgekleidet ist, die aus wärmeisolierendem Material besteht.

9. Trocken- und Sterilisationsschrank nach Anspruch 7 oder 8, dadurch gekennzeichnet, daß die äußere Wandung aus einem wärmeisolierenden Material besteht.

10. Trocken- und Sterilisationsschrank nach einem der Ansprüche 6, 7 oder 8, dadurch gekennzeichnet, daß ein Teil der Kühlluft über die Außenwände (z. B. Seiten-, Decken- und/oder Rückwandkanäle) angesaugt wird, um diese zu kühlen.

**Claims**

1. Process for drying and sterilizing items, in particular laundry, particularly delicate fabrics, pure wool, mattresses, cushions, duvet covers, woollen blankets and leather and any other objects, e.g. cutlery, surgical instruments, combs, scissors, brushes, blades, razors, in a drying and sterilizing cabinet, in which both the drying and sterilization of the, e.g. fabrics, is carried out in the same treatment chamber in a standing or hanging, mechanically unmoved state and sterilization is carried out directly after drying, characterised in that the air stream is conducted in a closed circuit consisting of an inner and an outer circut, and in that by a condensing and cooling aggregate, both systems are in contact in terms of energy, in that condensation is started by means of the condensing and cooling aggregate at the end of drying, and in that after sterilisation. the condensing and cooling aggregate is cooled.

2. Process according to claim 1, characterised in that condensation is carried out at a warm air temperature of below 100° C and sterilisation at a hot air temperature ranging from 121 to 130° C, preferably between 125 and 130° C.

3. Process according to clain 1 or 2, characterised in that condensation is carried out at a temperature of 45 to 60° C.

4. Process according to one of claims 1 to 3, characterised in that, during the sterilisation phase, the air stream of the inner circuit is severely reduced.

5. Process according to one of claims 1 to 3, characterised in that, during the sterilisation phase, the air stream of the outer circuit is not moved.

6. Drying and sterilising cabinet for carrying out a process according to one of claims 1 to 3, characterised in that the drying cabinet has two blowers driven separately by electric motors, one for the inner drying and sterilising circuit and one for the outer cooling circuit.

7. Drying and sterilising cabinet according to claim 6, characterised in that the inner wall consists of a heat-insulating material.

8. Drying and sterilising cabinet according to claim 6, characterised in that the whole interior is lined with an insulating layer, which consists of heat-insulating material.

9. Drying and sterilising cabinet according to claim 7 or 8, characterised in that the outer wall consists of a heat-insulating material.

10. Drying and sterilising cabinet according to one of claims 6, 7 or 8, characterised in that some of the cooling air is drawn out over the outer balls (e.g. side, top, and/or rear wall channels) in order to cool them.

**Revendications**

1. Procédé de séchage et de stérilisation d'objets, en particulier de linge lavé, en particulier de tissus fragiles, de laine pure, de couvre-pieds, de coussins, d' édredons, de couvertures de laine et de cuir, et d'autres objets à volonté, par exemple des couverts, des instruments médicaux, des peignes, des ciseaux, des brosses, des couteaux, des rasoirs, dans une armoire de séchage et de stérilisation, aussi bien le séchage que la stérilisation, par exemple, du tissu étant exécutés dans le même espace de traitement, dans l'état dressé ou suspendu, sans mouvement mécanique, et la stérilisation étant réalisée immédiatement après le séchage,
caractérisé en ce que
la courant d'air est guidé dans un circuit fermé constitué d'un circuit intérieur et d'un circuit extérieur, et en ce que les deux systèmes sont, en ce qui concerne l'énergie, en contact l'un avec l'autre à l'aide d'un ensemble de condensation et de refroidissement, en ce que la condensation est réglée au mcyen d'un ensemble de condensation et de refroidissement apreä la fin du séchorge et en ce que l'ensemble de condensation et de refroidissement refroidit après la stérilisation.

2. Procédé selon la revendication 1, caractérisé en ce que la condensation est effectuée avec une température d'air chaud inférieure à 190° C et la stérilisation avec une température d'air chaud comprise dans la zone entre 121 et 130° C, de préférence entre 125 et 130° C.

3. Procédé selon la revendication 1 ou 2, caractérisé en ce que la condensation est effectuée à une température de 45 à 60° C.

4. Procédé selon l'une des revendications 1 à 3, caractérisé en ce que le courant d'air du circuit intérieur est fortement réduit au cours de la phase de stérilisation.

5. Procédé selon l'une des revendications 1 à 3, caractérisé en ce que le courant d'air du circuit extérieur n'est pas mis en mouvement au cours de la phase de stérilisation.

6. Armoire de séchage et de stérilisation pour la mise en oeuvre de l'un des procédés selon i'un des revendications 1 à 5, caractérisée in ce que l'armoire de séchage comprend deux ventilateurs entrainés de façon séparée par des moteurs électriques, l'un pour le circuit intérieur de séchage et de stérilisation et un pour le circuit extérieur de refroidissement.

7. Armoire de séchage et de stérilisation selon la ravendication 6, caractérisée en ce que la paroi intérieure est en une matière thermiquement isolante.

8.  Armoire de séchage et de stérilisation selon la revendication 6, caractérisée en ce que tout le volume intérieur est revètu d'une couche isolante qui est en matière thermiquement isolante.

9.  Armoire de séchage et de stérilisation selon la revendication 7 ou 8 caractérisée en ce que la paroi extérieure est en matière thermiquement isolante.

10. Armoire de séchage et de stérilisation selon l'une des revendications 6, 7 ou 8, caractérisée en ce qu'une partie de l'air froid est aspirée par les parois extérieures (par exemple des canaux de parois latérales, supérieure et/ou arrière) pour refroidir celles-ci.

# Fig.1

EP 0 262 451 B1

- 150
- 100
- 50
- ≈ 20

°C

≈ 130°C

≈ 30°C

1    2    3

Gebläse des
externen
Kreislaufs

Ein

Aus

1    2    3

Gebläse des
internen
Kreislaufs

Schnell

Langsam

1    2    3

Zeit in h

Fig.2

Fig.3

Fig.4

Fig.5

Fig.6